# FASCICULE DE BREVET EUROPEEN

(11) **EP 0 850 634 B1**
(45) Date de publication et mention de la délivrance du brevet: **26.05.1999**
(21) Numéro de dépôt: 97402685.8
(22) Date de dépôt: 10.11.1997
(51) Int. Cl.: A61K 7/00, A61K 7/48

(54) **Système conservateur et son utilisation dans une composition cosmétique ou pharmaceutique**
Konservierungssystem und seine Verwendung in einer kosmetischen oder pharmazeutischen Zusammensetzung
Preservative system and its use in a cosmetic or pharmaceutical composition

(30) Priorité: 24.12.1996 FR 9615983
(43) Date de publication de la demande: 01.07.1998
(73) Titulaire: L'OREAL, 75008 Paris (FR)
(72) Inventeur: Bui-Bertrand, Lien, 91600 Savigny Sur Orge (FR); Carrel, Marie-Laure, 75007 Paris (FR)
(74) Mandataire: Tezier Herman, Béatrice

(56) Documents cités:
- EP-A- 0 117 135
- WO-A-94/27436
- WO-A-96/36310
- FR-A- 2 649 718
- US-A- 4 362 747
- US-A- 4 994 265
- P. ALEXANDER: "Preservatives in personal care products" SOAP, PERFUMERY & COSMETICS, vol. 60, no. 3, 1987, pages 47-49, XP002039063

## Description

L'invention se rapporte à un nouveau système conservateur comprenant l'association d'au moins un parahydroxybenzoate d'alkyle en C₁-C₄ avec du benzoate de sodium et du chlorure de N-(3-chloroallyl)-hexaminium. Elle se rapporte aussi à l'utilisation de ce nouveau système dans les domaines cosmétique ou pharmaceutique, et notamment dans des compositions destinées à être appliquées sur le visage et sur les yeux de mammifères et en particulier d'êtres humains. Ces compositions sont plus spécialement des lotions de nettoyage du visage et des yeux.

Il est courant d'introduire dans les compositions contenant de l'eau comme des compositions cosmétiques ou pharmaceutiques notamment dermatologiques, des conservateurs chimiques destinés en vue de lutter contre le développement et la prolifération des micro-organismes dans ces compositions. En effet, un tel développement de micro-organismes rendrait rapidement les compositions inaptes à l'utilisation. Pour éviter ce développement, il faut protéger les compositions à la fois contre les micro-organismes susceptibles de se développer à l'intérieur des compositions et contre ceux que l'utilisateur peut introduire dans celles-ci en les manipulant, en particulier lors de la préhension de produits en pot avec les doigts.

Les conservateurs chimiques les plus couramment utilisés dans les domaines ci-dessus sont notamment les parahydroxybenzoates d'alkyle en C₁-C₄ appelés ci-après parabens, ou des donneurs de formol. Malheureusement, ces conservateurs présentent l'inconvénient de causer des intolérances sur la peau humaine, telles que irritations et/ou allergies, et plus spécialement sur les peaux sensibles. Il en est de même pour les alcools ou les polyols, tels que l'éthanol ou le propylène glycol, notamment lorsqu'ils sont présents à des taux relativement importants. Ces problèmes d'intolérance se posent tout particulièrement lorsque les produits contenant ces conservateurs sont des produits de nettoyage et de démaquillage du visage et des yeux des personnes.

Par ailleurs, il est connu d'utiliser dans les produits de nettoyage de la peau et des yeux, et notamment dans les lotions démaquillantes pour les yeux, des conservateurs tels que le chlorure de benzalkonium, le chloroacétamide ou le thimerosal. Ainsi, le document WO-A-94/27436 enseigne un système conservateur comprenant un paraben et un composé d'ammonium quaternaire tel que le chlorure de benzalkonium ou le chlorure de cétylpyridinium. Toutefois, la tendance actuelle et la législation y afférente, évoluent de plus en plus vers la suppression de ces composés car ils ne sont pas totalement dépourvus de toxicité ou d'effets indésirables.

En outre, le document D&Cl, May 1986, 138 (5), p.50, 52-55, 61 décrit l'utilisation du chlorure de N-(3-chloroallyl)-hexaminium appelé ci-après Quaternium-15, comme conservateur cosmétique et ses associations, notamment avec les parabens. Toutefois, les systèmes décrits dans ce document présentent l'inconvénient de comporter des taux de Quaternium-15 trop importants (0,4 %), ce qui entraîne des inconforts oculaires (larmoiements, picotements, dessèchement, démangeaisons, rougeurs) ou des allergies.

On constate donc que subsiste le besoin d'un nouveau système conservateur ayant une action au moins aussi efficace que les systèmes de l'art antérieur, mais ne présentant pas leurs inconvénients et notamment non irritants pour la peau et les yeux.

La demanderesse a trouvé, de manière surprenante, qu'en associant le benzoate de sodium aux parabens et au Quaternium-15, on obtenait un système conservateur présentant une très bonne activité contre les micro-organismes et ne présentant aucun problème de toxicité tout en étant parfaitement bien toléré par la peau ou les yeux des personnes.

Aussi, la présente invention a pour objet un système conservateur comprenant au moins un parahydroxybenzoate d'alkyle en C₁-C₄ et du chlorure de N-(3-chloroallyl)-hexaminium, caractérisé en ce qu'il comprend en outre du benzoate de sodium.

Dans ce système conservateur, chacun des trois conservateurs est utilisé en une concentration plus faible que s'il était utilisé seul ou en présence d'autres conservateurs ; ceci va dans le sens d'une meilleure tolérance par la peau et les yeux. Ce système est de ce fait particulièrement approprié aux compositions topiques, et notamment cosmétiques et/ou dermatologiques, car il ne provoque ni intolérance ni rougeur ni picotements lors de l'application sur la peau ou les yeux tout en présentant une activité contre les micro-organismes au moins aussi efficace que celle des systèmes de l'art antérieur qui eux provoquent d'importants picotements.

Cette association contre les micro-organismes est efficace aussi bien sur les bactéries que les levures et moisissures, contaminants les plus gênants et les plus courants dans les compositions topiques ; elle est donc particulièrement intéressante à utiliser dans ce type de composition.

Aussi, l'invention a encore pour objet une composition cosmétique et/ou dermatologique, caractérisée en ce qu'elle contient le système conservateur tel que défini ci-dessus.

L'invention a encore pour objet l'utilisation du système conservateur tel que défini ci-dessus dans et/ou pour la préparation d'une composition cosmétique et/ou dermatologique pour limiter la prolifération microbienne.

L'invention a encore pour objet l'utilisation du système conservateur tel que défini ci-dessus dans et/ou pour la préparation d'une composition cosmétique et/ou dermatologique pour le démaquillage des yeux.

L'invention a encore pour objet l'utilisation du système conservateur tel que défini ci-dessus en tant qu'agent de lutte contre les micro-organismes dans et/ou pour la préparation d'une composition cosmétique et/ou dermatologique.

Le parahydroxybenzoate d'alkyle en C₁-C₄ peut être choisi en particulier parmi le parahydroxybenzoate de méthyle, appelé ci-après méthylparaben, le parahydroxybenzoate d'éthyle, appelé ci-après éthylparaben et le parahydroxybenzoate de propyle, appelé ci-après propylparaben. Selon un mode préféré de l'invention, il s'agit du méthylparaben.

Le système conservateur selon l'invention comprend de préférence un rapport en poids de la quantité de parahydroxybenzoate d'alkyle en C₁-C₄ sur la somme de la quantité de benzoate de sodium et de Quaternium-15 de 0,4 à 1,4.

La composition topique contenant le système conservateur selon l'invention comprend de 0,1 à 0,15 % en poids de paraben, de 0,1 à 0,2 % en poids de benzoate de sodium et de 0,01 à 0,03 % en poids de Quaternium-15 du poids total de la composition.

Les compositions de l'invention contiennent un milieu cosmétiquement et/ou dermatologiquement acceptable, c'est-à-dire compatible avec la peau, les muqueuses, les cheveux et le cuir chevelu. Elles peuvent comprendre les adjuvants classiquement mis en oeuvre dans les domaines considérés comme les corps gras, les solvants organiques, les agents solubilisants, les agents épaississants et gélifiants, les adoucissants (allantoïne), les antioxydants, les opacifiants, les agents stabilisants, les agents moussants, les parfums, les émulsionnants ioniques ou non, les charges, les séquestrants (EDTA disodique) et les chélateurs, les parfums, les filtres, les huiles essentielles, les matières colorantes, les pigments, les actifs hydrophiles ou lipophiles, les vésicules lipidiques encapsulant un ou plusieurs actifs ou tout autre ingrédient habituellement utilisé en cosmétique ou dermatologie. Elles peuvent contenir aussi des conservateurs autres que ceux appartenant au système revendiqué. Les quantités des différents constituants des compositions selon l'invention sont celles classiquement utilisées dans les domaines considérés. Bien entendu, ces adjuvants doivent être de nature et utilisés en quantité telle qu'ils ne perturbent pas le système conservateur selon l'invention.

Les compositions selon l'invention peuvent se présenter sous toutes les formes galéniques appropriées pour une application topique, notamment sous forme de solutions aqueuses, hydroalcooliques ou huileuses, de gels aqueux, hydroalcooliques ou huileux, de produits anhydres pâteux ou solides, d'émulsions obtenues par dispersion d'une phase grasse dans une phase aqueuse (H/E) ou inversement (E/H), de suspensions, de microémulsions, de microcapsules, de microparticules, ou encore de dispersions vésiculaires de type ionique (liposomes) et/ou non ionique. Dans les dispersions vésiculaires, les vésicules ne contiennent pas nécessairement un actif et servent à disperser et/ou à stabiliser une phase huileuse dans une phase aqueuse.

Les compositions de l'invention peuvent donc avoir l'aspect d'une crème, d'une lotion, d'un lait, d'un sérum, etc.. Les compositions selon l'invention peuvent être également utilisées sous forme de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (isoparaffine), les huiles d'origine végétale (huile d'amandes d'abricot, huile d'amande douce), les huiles d'origine animale, les huiles de synthèse (palmitate d'octyle, palmitate d'isopropyle), les huiles siliconées (cyclopentadiméthylsiloxane) et les huiles fluorées. On peut aussi utiliser comme matières grasses des alcools gras, des acides gras, des cires.

Comme émulsionnants, on peut citer par exemple le mélange de stéarate de glycéryle et de stéarate de PEG-100, vendu sous le nom d'Arlacel 165 par la société ICI, le mélange d'éthanolamides d'acide de coco (nom CTFA : Cocamide DEA), le cocoamphodiacétate disodique (nom CTFA : disodium cocoamphodiacetate), les polymères blocs oxyéthylénés oxypropylénés tels que le Poloxamer 184 (nom CTFA).

On peut y ajouter des agents tensioactifs tels que le mélange de Sodium Laureth Sulfate / Magnesium Laureth Sulfate / Sodium Laureth-8 Sulfate / Magnesium Laureth-8 Sulfate, vendu sous le nom de "Texapon ASV" par la société Henkel.

Comme gélifiants hydrophiles, on peut citer en particulier les polymères carboxyvinyliques (carbomer), les copolymères acryliques tels que les copolymères d'acrylates/alkylacrylates (Pemulen), les polyacrylamides, les polysaccharides, les gommes naturelles (gomme de xanthane) et les argiles, et, comme gélifiants lipophiles, on peut citer les argiles modifiées comme les bentones, les sels métalliques d'acides gras, la silice hydrophobe et les polyéthylènes.

Les compositions selon l'invention sont préparées selon les techniques bien connues de l'homme de l'art du domaine.

Ces compositions constituent notamment des produits de nettoyage et de démaquillage de la peau et des yeux, et en particulier des lotions nettoyantes et démaquillantes.

Aussi, la présente invention a aussi pour objet une lotion de nettoyage et/ou de démaquillage de la peau et/ou des yeux, caractérisée en ce qu'elle contient le système conservateur tel que défini ci-dessus.

La lotion selon l'invention peut comprendre notamment comme adjuvants au moins un tensioactif non-ionique et/ou anionique. Par ailleurs, elle peut comprendre aussi au moins un glycol tel que le propylène glycol, le butylène glycol et l'hexylène glycol.

Dans tout ce qui suit, les pourcentages sont donnés en poids sauf mention contraire.

D'autre caractéristiques et avantages de l'invention pourront apparaître dans les exemples qui suivent, qui sont donnés à titre purement illustratif et nullement limitatif.

Le test suivant met en évidence l'activité de l'association selon l'invention sur les micro-organismes.

Les étapes pour réaliser ce test sont les suivantes :
1) Culture du micro-organisme des souches utilisées :
   *Escherichia coli* (bactérie)
   *Pseudomonas aeruginosa* (bactérie)
   *Enterococcus faecalis* (bactérie)
   *Candida albicans* (levure)
   *Aspergillus niger* (moisissure)

   Les souches sont préparées dans le milieu de culture tryptone-sel.
2) Préparation de l'inoculum : L'inoculum est constitué d'une culture de 24 heures du germe-test sur un milieu nutritif gélosé approprié et dilué de façon à obtenir une suspension contenant 10⁵ à 10⁶ germes/ml. Un dénombrement de 5 inocula est effectué.
3) Préparation de l'échantillon : On dépose dans 5 flacons de verre appelés piluliers , 20 g du produit à tester.
4) Inoculation : on introduit 0,2 ml d'inoculum de chaque souche dans un pilulier différent et on homogénéise. Les piluliers sont alors mis à incuber à 22°C pendant 7 jours.
5) Prélèvements et dénombrements :
   Après les 7 jours d'incubation, 1 g de produit est prélevé de chacun des piluliers. Des dilutions décimales sont effectuées dans un diluant renfermant des inhibiteurs de conservateurs qui vont stopper l'action des conservateurs. Ces dilutions sont étalées en surface de boites de Pétri gélosées, mises ensuite à
incuber à 35°C pendant 72 heures pour les bactéries et levures et pendant 5 jours pour les moisissures. Les colonie apparues sont alors dénombrées en tenant compte des dilutions.

### Exemple 1 : système conservateur

Le système testé a la composition suivante :

| | |
|---|---|
| - Méthylparaben | 0,15 % |
| - Quaternium-15 | 0,015 % |
| - Benzoate de sodium | 0,13 % |

Les résultats obtenus sont rassemblés dans le tableau 1.

**Tableau 1**

| Germes | Inoculum germes/gramme | Germes survivants à 7 jours |
|---|---|---|
| *Escherichia coli* | 3,4.10⁶ | < 200* |
| *Pseudomonas aeruginosa* | 5,3.10⁶ | < 200* |
| *Enterococcus faecalis* | 3,4,10⁶ | 4.10⁴ |
| *Candida albicans* | 3,5.10⁶ | < 200* |
| *Aspergillus niger* | 1,2.10⁶ | 1,4.10³ |

| | | |
|---|---|---|
| *Seuil de sensibilité de la méthode | | |

Les résultats montrent une diminution d'au moins 2 log des germes, ce qui représente la quantité satisfaisante.

### Exemple 2 : Système conservateur

Le système testé a la composition suivante :

| | |
|---|---|
| - Méthylparaben | 0,1 % |
| - Quaternium-15 | 0,018 % |
| - Benzoate de sodium | 0,1 % |

Les résultats obtenus sont rassemblés dans le tableau 2.

**Tableau 2**

| Germes | lnoculum germes/gramme | Germes survivants à 7 jours |
|---|---|---|
| *Escherichia coli* | 2,5.10⁶ | < 200* |
| *Pseudomonas aeruginosa* | 3,1.10⁶ | < 200* |
| *Enterococcus faecalis* | 2,1.10⁶ | < 200* |
| *Candida albicans* | 3,1.10⁶ | < 200* |
| *Aspergillus niger* | 4.10⁶ | 5,8.10³ |

| | | |
|---|---|---|
| *Seuil de sensibilité de la méthode | | |

Le tableau montre que le système selon l'invention donne de très bons résultats : il n'y a plus de germes détectables pour les bactéries et levure, et la quantité de germes des moisissures est fortement diminuée.

### Exemple 3 : lait démaquillant

| | |
|---|---|
| - Palmitate d'isopropyle | 5 % |
| - Huile d'amandes d'abricot | 0,25 % |
| - Huile d'amande douce | 0,25 % |
| - Methylparaben | 0,15 % |
| - Quaternium-15 | 0,025 % |
| - Benzoate de sodium | 0,1 % |
| - EDTA disodique | 0,05 % |
| - Hydroxyde de sodium | 0,1 % |
| - Carbomer | 0,25 % |
| - Gomme de xanthane | 0,1 % |
| - Glycérine | 3 % |
| - Cocamide DEA | 0,5 % |
| - Arlacel 165 | 1,7 % |
| - Eau déminéralisée qsp | 100 % |

Ce lait permet de démaquiller le visage sans irritation.

### Exemple 4 : eau démaquillante

| | |
|---|---|
| - Methylparaben | 0,1 % |
| - Quaternium-15 | 0,025 % |
| - Benzoate de sodium | 0,2 % |
| - Parfum | 0,04 % |
| - Hydroxyde de sodium | 0,025 % |
| - EDTA disodique | 0,05 % |
| - Poloxamer 184 | 2 % |
| - Eau déminéralisée qsp | 100 % |

Cette eau démaquillante peut être utilisée pour le démaquillage du visage et des yeux en toute sécurité, c'est-à-dire sans provoquer d'inconfort oculaire.

### Exemple 5 : lotion démaquillante

| | |
|---|---|
| - Allantoïne | 0,05 % |
| - Methylparaben | 0,15 % |
| - Quaternium-15 | 0,018 % |
| - Benzoate de sodium | 0,1 % |
| - Parfum | 0,01 % |
| - Chlorure de sodium | 0,9 % |
| - EDTAdisodique | 0,05 % |
| - Propylène glycol | 1 % |
| - Disodium cocoamphodiacetate | 2 % |
| - Texapon ASV | 0,5 % |
| - Eau déminéralisée qsp | 100 % |

Cette lotion démaquillante permet de démaquiller les yeux en tout confort, c'est-à-dire sans engendrer d'inconfort oculaire.

### Exemple 6 : démaquillant deux en un

| | |
|---|---|
| - Palmitate d'octyle | 15 % |
| - Methylparaben | 0,15 % |
| - Quaternium-15 | 0,025 % |
| - Benzoate de sodium | 0,1 % |
| - EDTA disodique | 0,1 % |
| - Triéthanolamine | 0,82 % |
| - Pemulen | 0,15 % |
| - Carbomer | 0,42 % |
| - Glycérine | 5 % |
| - Texapon ASV | 0,9 % |
| - Disodium cocoamphodiacetate | 0,5 % |
| - Eau déminéralisée qsp | 100 % |

Ce démaquillant peut être utilisé aussi bien pour le visage que pour les yeux et est très confortable à utiliser.

## Revendications

1. Système conservateur comprenant au moins un parahydroxybenzoate d'alkyle en C₁-C₄ et du chlorure de N-(3-chloroallyl)-hexaminium, caractérisé en ce qu'il comprend en outre du benzoate de sodium.

2. Système conservateur selon la revendication 1, caractérisé en ce que le parahydroxybenzoate d'alkyle en C₁-C₄ est choisi parmi le parahydroxybenzoate de méthyle, le parahydroxybenzoate d'éthyle ou le parahydroxybenzoate de propyle.

3. Système conservateur selon la revendication précédente, caractérisé en ce que le le parahydroxybenzoate est le parahydroxybenzoate de méthyle.

4. Système conservateur selon l'une quelconque des revendications précédentes, caractérisé en ce qu'il comprend un rapport en poids de la quantité de parahydroxybenzoate d'alkyle en C₁-C₄ sur la somme de la quantité de benzoate de sodium et de chlorure de N-(3-chloroallyl)-hexaminium de 0,4 à 1,4.

5. Composition cosmétique et/ou dermatologique, caractérisée en ce qu'elle contient le système conservateur selon les revendications 1 à 4.

6. Composition selon la revendication précédente, caractérisée en ce qu'elle comprend 0,1 à 0,15 % en poids de parahydroxybenzoate d'alkyle en C₁-C₄, 0,1 à 0,2 % en poids de benzoate de sodium et 0,01 à 0,03 % en poids de chlorure de N-(3-chloroallyl)-hexaminium du poids total de la composition.

7. Composition selon la revendication 5 ou 6, caractérisée en ce qu'elle constitue une composition de nettoyage et/ou de démaquillage.

8. Composition selon la revendication précédente, caractérisée en ce qu'elle constitue une composition de démaquillage des yeux.

9. Composition selon l'une quelconque des revendications 5 à 8, caractérisée en ce qu'elle se présente sous forme d'une solution aqueuse ou hydroalcoolique, d'une émulsion, d'une microémulsion, d'un gel aqueux ou anhydre, d'une dispersion de vésicules.

10. Composition selon l'une quelconque des revendications 5 à 9, caractérisée en ce qu'elle se présente sous forme d'une lotion.

11. Composition selon l'une quelconque des revendications 5 à 10, caractérisée en ce qu'elle comprend, en outre, au moins un adjuvant choisi parmi parmi les corps gras, les solvants organiques, les agents solubilisants, les gélifiants, les agents moussants, les émulsionnants, les charges, les pigments, les actifs hydrophiles ou lipophiles, les vésicules lipidiques.

12. Lotion de nettoyage et/ou de démaquillage de la peau et/ou des yeux, caractérisée en ce qu'elle contient le système conservateur selon l'une quelconque des revendications 1 à 4.

13. Lotion selon la revendication 12, caractérisée en ce qu'elle comporte en outre au moins un tensioactif non ionique et/ou anionique.

14. Lotion selon la revendication 12 ou 13, caractérisée en ce qu'elle comprend en outre au moins un glycol.

15. Utilisation du système conservateur selon l'une quelconque des revendications 1 à 4 dans et/ou pour la préparation d'une composition cosmétique et/ou dermatologique pour limiter la prolifération microbienne.

16. Utilisation du système conservateur selon l'une quelconque des revendications 1 à 4 dans et/ou pour la préparation d'une composition cosmétique et/ou dermatologique pour le démaquillage des yeux.

17. Utilisation du système conservateur selon l'une quelconque des revendications 1 à 4 en tant qu'agent de lutte contre les micro-organismes dans et/ou pour la préparation d'une composition cosmétique et/ou dermatologique.

## Claims

1. Preserving system comprising at least one C₁-C₄alkyl para-hydroxybenzoate and N-(3-chloroallyl)-hexaminium chloride, characterized in that it also comprises sodium benzoate.

2. Preserving system according to Claim 1, characterized in that the C₁-C₄alkyl para-hydroxybenzoate is chosen from methyl para-hydroxybenzoate, ethyl para-hydroxybenzoate and propyl para-hydroxybenzoate.

3. Preserving system according to the preceding claim, characterized in that the para-hydroxybenzoate is methyl para-hydroxybenzoate.

4. Preserving system according to any one of the preceding claims, characterized in that it comprises a weight ratio of the amount of C₁-C₄alkyl parahydroxybenzoate to the sum of the amount of sodium benzoate and N-(3 chloroallyl)hexaminium chloride of 0.4 to 1.4.

5. Cosmetic and/or dermatological composition, characterized in that it contains the preserving system according to Claims 1 to 4.

6. Composition according to the preceding claim, characterized in that it comprises 0.1 to 0.15% by weight of C₁-C₄alkyl para-hydroxybenzoate, 0.1 to 0.2% by weight of sodium benzoate and 0.01 to 0.03% by weight of N-(3-chloroallyl)hexaminium chloride relative to the total weight of the composition.

7. Composition according to Claim 5 or 6, characterized in that it constitutes a cleansing and/or make-up-removing composition.

8. Composition according to the preceding claim, characterized in that it constitutes a composition for removing make-up from around the eyes.

9. Composition according to any one of Claims 5 to 8, characterized in that it is in the form of an aqueous or aqueous-alcoholic solution, an emulsion, a microemulsion, an aqueous or anhydrous gel, or a vesicle dispersion.

10. Composition according to any one of Claims 5 to 9, characterized in that it is in the form of a lotion.

11. Composition according to any one of Claims 5 to 10, characterized in that it also comprises at least one adjuvant chosen from fatty substances, organic solvents, solubilizing agents, gelling agents, foaming agents, emulsifiers, fillers, pigments, hydrophilic or lipophilic active agents and lipid vesicles.

12. Cleansing and/or make-up-removing lotion for the skin and/or the eyes, characterized in that it contains the preserving system according to any one of Claims 1 to 4.

13. Lotion according to Claim 12, characterized in that it also contains at least one nonionic and/or anionic surfactant.

14. Lotion according to Claim 12 or 13, characterized in that it also comprises at least one glycol.

15. Use of the preserving system according to any one of Claims 1 to 4, in and/or for the preparation of a cosmetic and/or dermatological composition for limiting microbial proliferation.

16. Use of the preserving system according to any one of Claims 1 to 4, in and/or for the preparation of a cosmetic and/or dermatological composition for removing make-up from around the eyes.

17. Use of the preserving system according to any one of Claims 1 to 4, as an agent for combating micro-organisms, in and/or for the preparation of a cosmetic and/or dermatological composition.

## Patentansprüche

1. Konservierungssystem, das mindestens ein C₁₋₄-Alkyl-p-hydroxybenzoat und N-(3-Chlorallyl)-hexaminiumchlorid enthält, dadurch gekennzeichnet, daß es ferner Natriumbenzoat enthält.

2. Konservierungssystem nach Anspruch 1, dadurch gekennzeichnet, daß das C₁₋₄-Alkyl-p-hydroxybenzoat unter Methyl-p-hydroxybenzoat, Ethyl-p-hydroxybenzoat oder Propyl-p-hydroxybenzoat ausgewählt ist.

3. Konservierungssystem nach dem vorhergehenden Anspruch, dadurch gekennzeichnet, daß das p-Hydroxybenzoat Methyl-p-hydroxybenzoat ist.

4. Konservierungssystem nach einem der vorhergehenden Ansprüche, dadurch gekennzeichnet, daß das Gewichtsverhältnis von C₁₋₄-Alkyl-p-hydroxybenzoat zu der Summe von Natriumbenzoat und N-(3-Chlorallyl)-hexaminiumchlorid in dem Konservierungssystem im Bereich von 0,4 bis 1,4 liegt.

5. Kosmetische und/oder dermatologische Zusammensetzung, dadurch gekennzeichnet, daß sie das Konservierungssystem nach den Ansprüchen 1 bis 4 enthält.

6. Zusammensetzung nach dem vorhergehenden Anspruch, dadurch gekennzeichnet, daß sie 0,1 bis 0,15 Gew.-% C₁₋₄-Alkyl-p-hydroxybenzoat, 0,1 bis 0,2 Gew.-% Natriumbenzoat und 0,01 bis 0,03 Gew.-% N-(3-Chlorallyl)-hexaminiumchlorid, bezogen auf das Gesamtgewicht der Zusammensetzung, enthält.

7. Zusammensetzung nach Anspruch 5 oder 6, dadurch gekennzeichnet, daß es sich um eine Zusammensetzung zum Reinigen und/oder zum Abschminken handelt,

8. Zusammensetzung nach dem vorhergehenden Anspruch, dadurch gekennzeichnet, daß es sich um eine Zusammensetzung zum Abschminken der Augen handelt.

9. Zusammensetzung nach einem der Ansprüche 5 bis 8, dadurch gekennzeichnet, daß sie in Form einer wässerigen oder wässerig-alkoholischen Lösung, einer Emulsion, einer Mikroemulsion, eines wässerigen oder wasserfreien Gels oder einer Vesikeldispersion vorliegt.

10. Zusammensetzung nach einem der Ansprüche 5 bis 9, dadurch gekennzeichnet, daß sie in Form einer Lotion vorliegt.

11. Zusammensetzung nach einem der Ansprüche 5 bis 10, dadurch gekennzeichnet, daß sie ferner mindestens einen Zusatzstoff enthält, der ausgewählt ist unter Fettsubstanzen, organischen Lösemitteln, Lösungsvermittlern, Gelbildnern, Schaumbildnern, Emulgatoren, Füllstoffen, Pigmenten, hydrophilen oder lipophilen Wirkstoffen und Lipidvesikeln.

12. Lotion zum Reinigen und/oder Abschminken der Haut und/oder der Augen, dadurch gekennzeichnet, daß sie das Konservierungssystem nach einem der Ansprüche 1 bis 4 enthält.

13. Lotion nach Anspruch 12, dadurch gekennzeichnet, daß sie ferner mindestens einen nichtionischen und/oder anionischen grenzflächenaktiven Stoff enthält.

14. Lotion nach Anspruch 12 oder 13, dadurch gekennzeichnet, daß sie ferner mindestens ein Glykol enthält.

15. Verwendung des Konservierungssystems nach einem der Ansprüche 1 bis 4 in einer kosmetischen und/oder dermatologischen Zusammensetzung und/oder zu deren Herstellung, um die Mikrobenvermehrung einzuschränken.

16. Verwendung des Konservierungssystems nach einem der Ansprüche 1 bis 4 in einer kosmetischen und/oder dermatologischen Zusammensetzung zum Abschminken der Augen und/oder zu deren Herstellung.

17. Verwendung des Konservierungssystems nach einem der Ansprüche 1 bis 4 als Mittel zur Bekämpfung von Mikroorganismen in einer kosmetischen und/oder dermatologischen Zusammensetzung und/oder zu deren Herstellung.
